# EUROPEAN PATENT APPLICATION

(11) **EP 4 089 118 A1**
(43) Date of publication of application: **16.11.2022**
(21) Application number: 21738228.2
(22) Date of filing: 08.01.2021
(51) Int. Cl.: C07K 19/00, A61K 38/17, A61P 25/00, C07K 14/47, C12N 5/10, C12N 15/12, C12N 15/62, C12P 21/02

(54) **ARTIFICIAL EXCITATORY SYNAPSE CONNECTOR AND USE THEREOF FOR SPINAL CORD INJURY**

(30) Priority: 10.01.2020 JP 2020002879
(71) Applicant: Keio University, Tokyo, 108-8345 (JP); Aichi Medical University, Aichi 480-1195 (JP)
(72) Inventor: YUZAKI, Michisuke, Tokyo 160-8582 (JP); SUZUKI, Kunimichi, Tokyo 160-8582 (JP); TAKEUCHI, Kosei, Nagakute-shi, Aichi 480-1195 (JP); ARICESCU, Alexandru Radu, Cambridge Biomedical Campus, Cambridge CB2 0QH (GB)
(74) Representative: Gill Jennings & Every LLP
(86) International application number: PCT/JP2021/000540
(87) International publication number: WO 2021/141118

(57) **Abstract**

The present invention provides multimers of one or more fusion proteins, each fusion protein including a Nrx-binding region of a Cblnl protein, a multimerization domain, and a AMPA receptor-binding region of a Nptx1 protein, and pharmaceutical compositions containing the multimer(s) for use in forming new synaptic connections between excitatory interneurons.

## Description

### Technical field

The present invention relates to artificial excitatory synaptic connectors and their use in the treatment of spinal cord injuries.

### Background art

Cblnl is a secreted protein belonging to the Clq family and is mainly produced in and secreted from the cerebellar granule cells (Non-patent literature 1). Cbln1 binds to the presynaptic neurexin (Nrx). Cbln 1 is crucial for synapse formation between the parallel fibers and Purkinje cells (parallel fiber synapses) (Non-patent literatures 2-4). The Cbln1 knockout (cbln1-/-) mice displayed reduced parallel-fiber synapse density and severe cerebellar ataxia symptoms (Non-patent literatures 1 and 5).

Furthermore, the fusion proteins that connect the Nrx-binding element of a Cblnl protein and the AMPA-type glutamate receptor-binding element of a Nptxl protein via a trimerization domain increased synapses between parallel cerebellar fibers and Purkinje cells and enhanced electrophysiological synaptic responses in mice (cbln1-/-, GluD2-/-) with cerebellar ataxia. The fusion proteins were also reported to recover the aforementioned cerebellar ataxia symptoms (Non-patent literature 6).

### Related art documents

### Non-patent literature

Non-patent literature 1: Hirai, H., Pang, Z., Bao, D. et al.: Cblnl is essential for synaptic integrity and plasticity in the cerebellum. Nat. Neurosci., 8, 1534-1541 (2005);
Non-patent literature 2: Matsuda, K., Miura, E., Miyazaki, T. et al.: Cblnl is a ligand for an orphan glutamate receptor δ2, a bidirectional synapse organizer. Science, 328, 363-368 (2010);
Non-patent literature 3: Matsuda, K. & Yuzaki, M.: Cbln family proteins promote synapse formation by regulating distinct neurexin signaling pathways in various brain regions. Eur. J. Neurosci., 33, 1447-1461 (2011);
Non-patent literature 4: Uemura, T., Lee, S. J., Yasumura, M. et al.: Trans-synaptic interaction of GluRδ2 and Neurexin through Cblnl mediates synapse formation in the cerebellum. Cell, 141, 1068-1079 (2010);
Non-patent literature 5: Ito-Ishida, A., Miura, E., Emi, K. et al.: Cblnl regulates rapid formation and maintenance of excitatory synapses in mature cerebellar Purkinje cells in vitro and in vivo. J. Neurosci., 28, 5920-5930 (2008); and
Non-patent literature 6: Suzuki, K., et al., Fourth Annual Conference of COST Action ECMNET, October 1st, 2014.

### Summary of the invention

The present invention provides a multimer of one or more fusion proteins, each fusion protein containing a Nrx-binding region of a Cblnl protein, a multimerization domain, and a AMPA receptor-binding region of Nptxl, and provides pharmaceutical compositions containing the multimer for use in forming new synaptic connections between excitatory interneurons (especially between excitatory interneurons in the spinal cord).

The present inventors found that a fusion protein (artificial excitatory synaptic connector) that can connect Nrx expressed in presynaptic cells and AMPA-type glutamate receptors (AMPARs) expressed in postsynaptic cells formed new connection between excitatory interneurons in the spinal cord, and that, more surprisingly, the fusion protein healed spinal cord injuries and recovered motor function. This restoration of motor function through administration of the artificial excitatory synaptic connector was also effective for spinal cord injuries at subacute and chronic phases. The present invention is based on these findings.

According to the present invention, the following inventions are provided by way of examples.
[1] A multimer (preferably a hexamer) of one or more fusion proteins,
   each fusion protein containing a Nrx-binding region of a Cblnl protein, a trimerization domain, and a AMPA receptor-binding region of a Nptxl protein, wherein
   two trimers of the fusion protein are linked to each other by a disulfide bond.
[2] A pharmaceutical composition including the multimer (preferably a hexamer) of the one or more fusion proteins according to [1] above.
[3] A pharmaceutical composition for use in forming a new synaptic connection between excitatory interneurons,
   the pharmaceutical composition including a fusion protein, the fusion protein containing a Nrx-binding region of a Cbln1 protein, a multimerization domain, and a AMPA receptor-binding region of a Nptx1 protein.
[4] The pharmaceutical composition according to [3] above, wherein the excitatory interneurons are those in the spinal cord.
[5] The pharmaceutical composition according to [3] or [4] above for use in treating a spinal cord injury.
[5A] A pharmaceutical composition for use in treating a spinal cord injury,
   the pharmaceutical composition including a multimer of one or more fusion proteins, each fusion protein containing a Nrx-binding region of a Cblnl protein, a multimerization domain, and a AMPA receptor-binding region of Nptx1.
[6] The pharmaceutical composition according to any one of [3] to [5] and [5A] above, wherein the multimerization domain is a trimerization domain.
[7] The pharmaceutical composition according to any one of [3] to [5], [5A] and [6] above, wherein the multimer of the one or more fusion proteins is a hexamer.
[8] The pharmaceutical composition according to any one of [2] to [7] above, wherein the pharmaceutical composition is a therapeutic agent for a spinal cord injury.
[9] A method for manufacturing a hexamer of the one or more fusion proteins according to [1] above or a pharmaceutical composition containing the hexamer, the method including:
   culturing cells having a nucleic acid having a signal sequence and encoding a fusion protein of the present invention under a condition suitable for protein expression;
   acquiring a culture supernatant; and
   collecting the hexamer of the one or more fusion proteins from the acquired culture supernatant.
[1A] A multimer of one or more fusion proteins,
   each fusion protein containing a Nrx-binding region of a Cblnl protein, a multimerization domain, and a AMPA receptor-binding region of a Nptxl protein.
[2A] The multimer according to [1A] above, wherein the multimerization domain is a trimerization domain, and the multimer is a hexamer consisting of two trimers of the fusion protein, the trimers being linked to each other by a disulfide bond.
[3A] The multimer according to [1A] or [2A] above, wherein the Nrx-binding region of the Cblnl protein is capable of binding to Nrx containing splice site 4 (i.e., Nrx(S4+)).
[4A] The multimer according to any one of [1A] to [3A] above, wherein the AMPA-type glutamate receptor-binding region of Nptxl corresponds to a pentraxin domain of human neuropentraxin-1.
[5A] The multimer according to any one of [1A] to [4A] above, wherein the multimerization domain is interposed between the Nrx-binding region of the Cblnl protein and the AMPA-type glutamate receptor-binding region of Nptx1, or is sandwiched between the two regions.
[6A] The multimer according to any one of [1A] to [5A] above, wherein the Nrx-binding region of the Cblnl protein and the multimerization domain are connected to each other via a linker and/or the multimerization domain and the AMPA receptor-binding region of Nptxl are connected to each other via a linker.
[7A] The multimer according to [6A] above, wherein the linker has one of a sequence of SEQ ID NOs. 3-9.
[8A] A nucleic acid encoding a fusion protein as defined in any one of [1A] to [5A] above, the nucleic acid having a nucleotide sequence of SEQ ID NO. 1.
[9A] The multimer according to any one of [1A] to [5A] above, wherein the fusion protein has an amino acid sequence of SEQ ID NO. 2.
[10A] A pharmaceutical composition including the multimer according to any one of [1A] to [9A] above.
[11A-1] A pharmaceutical composition for use in forming a new synaptic connection between excitatory interneurons,
   the pharmaceutical composition including a fusion protein containing a neurexin (Nrx)-binding region of a cerebellin-1 (Cbln1 protein), a multimerization domain, and a AMPA receptor-binding region of a neuronal pentraxin-1 (Nptxl) protein.
[11A-2] A pharmaceutical composition for use in forming a new synaptic connection between excitatory interneurons,
   the pharmaceutical composition including a multimer of one or more fusion proteins, each fusion protein containing a neurexin (Nrx)-binding region of acerebellin-1 (Cbln1 protein), a multimerization domain, and a AMPA receptor -binding region of a neuronal pentraxin-1 (Nptxl) protein.
[11A-3] A pharmaceutical composition for use in forming a new synaptic connection between excitatory interneurons, the pharmaceutical composition including a multimer of one or more fusion proteins, each fusion protein containing a neurexin (Nrx)-binding region of a cerebellin-1 (Cbln1 protein), a multimerization domain, and a AMPA receptor -binding region of a neuronal pentraxin-1 (Nptxl) protein, the multimerization domain being a trimerization domain, and the multimer being a hexamer consisting of two trimers of the fusion protein, the trimers being linked to each other by a disulfide bond.

Hereinafter, [11A-1], [11A-2], and [11A-3] are collectively referred to as [11A] in [12A] and the items that follow.

[12A] The pharmaceutical composition according to [10A] or [11A] above, wherein the excitatory interneurons are those in the spinal cord.

[13A] The pharmaceutical composition according to any one of [10A] to [12A] above for use in treating a spinal cord injury.

[14A] The pharmaceutical composition according to any one of [10A] to [13A] above, wherein the multimerization domain is a trimerization domain.

[15A] The pharmaceutical composition according to any one of [10A] to [14A] above, wherein the multimerization domain is a trimerization domain, and the multimer is a hexamer consisting of two trimers of the fusion protein linked to each other by a disulfide bond.

[16A] The pharmaceutical composition according to any one of [10A] to [15A] above, wherein the pharmaceutical composition is a therapeutic agent for a spinal cord injury.

[17A] A method for manufacturing the pharmaceutical composition according to any one of [10A] to [15A] above, the method including:
culturing a cell having a nucleic acid having a signal sequence and encoding a fusion protein of the present invention under a condition suitable for protein expression;
acquiring a culture supernatant; and
collecting a multimer of one or more fusion proteins from the acquired culture supernatant.

[18A] The method according to [17A] above, wherein the multimerization domain is a trimerization domain, and the multimer is a hexamer consisting of two trimers of the fusion protein, the trimers being linked to each other by a disulfide bond.

[0010] [19A] A conjugate including a first molecule that binds to neurexin (Nrx) and a second molecule that binds to neuronal pentraxin-1 (Nptxl), wherein the first and second molecules are each selected from the group consisting of an antibody, an antigen-binding fragment thereof, and an aptamer.

[20A] A pharmaceutical composition including the conjugate according to [19A] above.

[21A] The pharmaceutical composition according to [20A] above for use in forming a new synaptic connection between excitatory interneurons.

[22A] The pharmaceutical composition according to [21A] above, wherein the excitatory interneurons are those in the spinal cord.

[23A] The pharmaceutical composition according to [20A] above for use in treating a spinal cord injury.

[24A] The pharmaceutical composition according to [20A] above, wherein the pharmaceutical composition is a therapeutic agent for a spinal cord injury.

### Brief description of the drawings

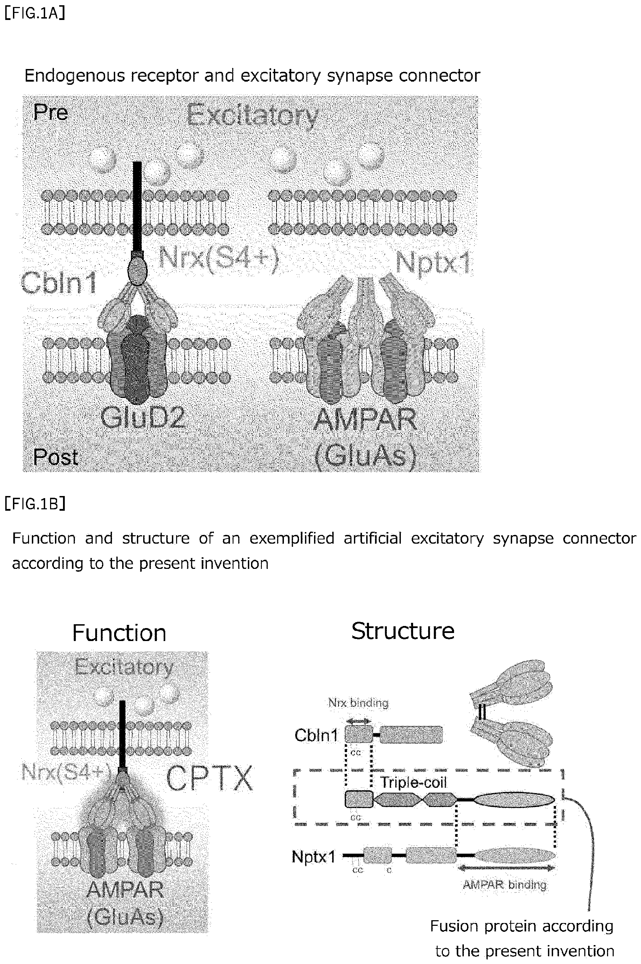

[Fig. 1A] Fig. 1A is a schematic diagram including and showing natural presynaptic and postsynaptic cells. In natural synapses, Cblnl secreted between synapses links Nrx(S4+) expressed in the presynaptic cells and glutamate D2 receptors (GluD2) expressed in the postsynaptic cells. Nptxl only binds to the AMPA-type glutamate receptors (AMPARs) expressed in the postsynaptic cells but not to the presynaptic cells.
[Fig. 1B] Fig. 1B shows a schematic diagram of a structure of an exemplified fusion protein (chimeric protein) of the present invention and its anticipated function. The illustrated fusion protein of the present invention contains a trimerization domain between a Nrx-binding domain of a Cblnl protein and an AMPAR-binding domain of a Nptxl protein (see the structure in Fig. 1B). The fusion protein (chimeric protein) of the present invention can link Nrx(S4+) in the presynaptic cell and the AMPAR in the postsynaptic cell in the synaptic cleft and transmit excitatory signals from the presynaptic cell to the postsynaptic cell (see the function in Fig. 1B). Therefore, the fusion protein of the present invention can act as an artificial excitatory synaptic connector.

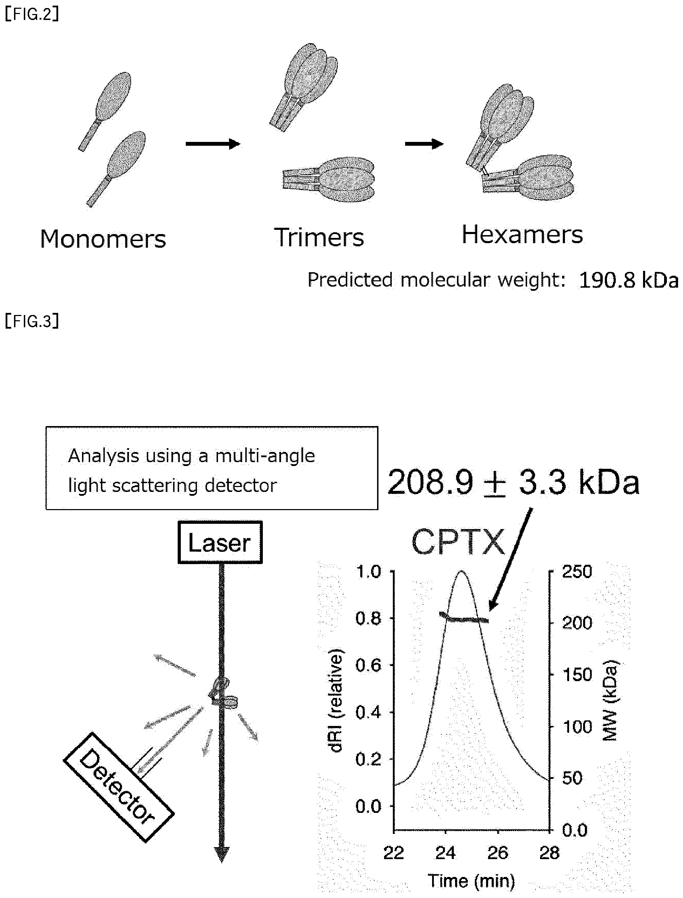

[Fig. 2] Fig. 2 shows that an exemplified fusion protein of the present invention forms a trimer and two trimers can assemble into a hexamer.
[Fig. 3] Fig. 3 shows the results of a multi-angle light scattering analysis of fusion proteins of the present invention prepared in the Example. According to the analysis results, the fusion proteins of the present invention were estimated to have a molecular weight of 208.9 ± 3.3 kDa. This estimated molecular weight corresponds to the theoretically predicted molecular weight in Fig. 2, suggesting that the fusion proteins of the present invention prepared in the Example form a hexamer.

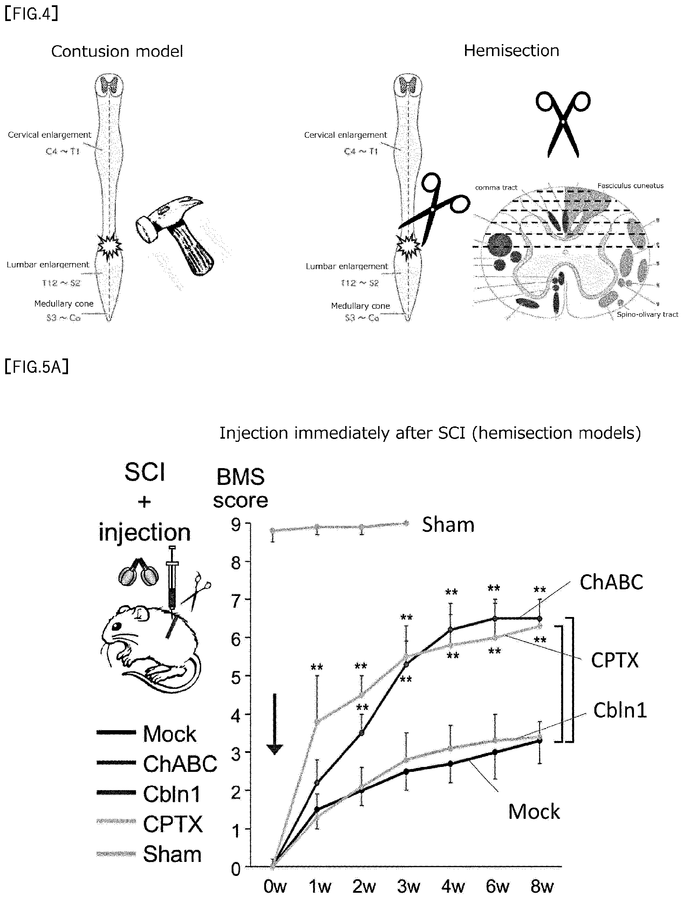

[Fig. 4] Fig. 4 is a schematic representation of how the contusion (compression) and hemisection (SCI) models of mice were prepared.
[Fig. 5A] Fig. 5A shows time-dependent changes in Basso Mouse Scale (BMS) scores after injecting a fusion protein of the present invention into the spinal cord of the hemisection models immediately after the hemisection. In the figure, "Mock" represents the negative control group that received HEPES buffer after SCI; "ChABC" is an enzyme that digests chondroitin sulfate glycosaminoglycans (chondroitinase ABC) having therapeutic effects on spinal cord injuries; "Cblnl" represents the full length of the human Cblnl protein; and "Sham" represents the group that did not develop any spinal cord injury after myelotomy. In the figure, "w" denotes week(s). Note that unlike CPTX, Cblnl cannot form a complex of Nrx(S4+) and AMPA receptors due to the absence of a binding element to AMPA receptors.

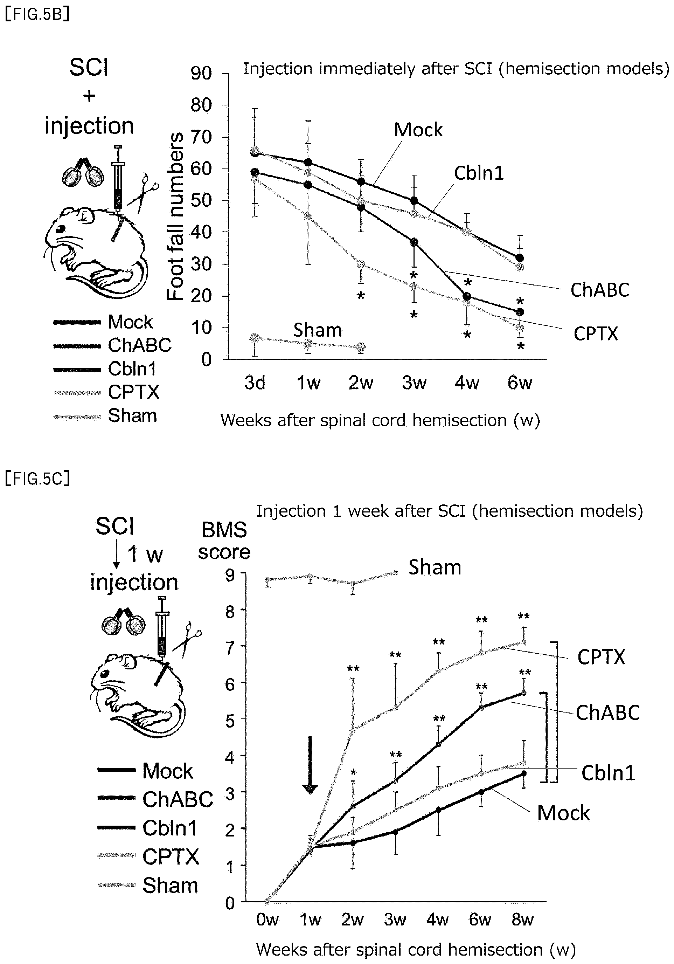

[Fig. 5B] Fig. 5B shows the results of footfall test overtime after injecting a fusion protein of the present invention into the spinal cord of the hemisection models immediately after the hemisection. Mice were placed on a wire-mesh grid and videotaped for 5 minutes while on the grid. The number of footfalls from the grid was counted for mice that walked at least 70 times on the grid for 3 minutes. In the figure, "Mock" represents the negative control group that received HEPES buffer after SCI; "ChABC" is an enzyme that digests chondroitin sulfate glycosaminoglycans (chondroitinase ABC) having therapeutic effects on spinal cord injuries; "Cblnl" represents the full length of the human Cblnl protein; and "Sham" represents the group in which any spinal cord injury was not caused after myelotomy. In the figure, "w" denotes week(s). Note that unlike CPTX, Cblnl cannot form a complex of Nrx(S4+) and AMPA receptors due to the absence of a binding element to AMPA receptors.
[Fig. 5C] Fig. 5C shows time-dependent changes in Basso Mouse Scale (BMS) scores after injecting a fusion protein of the present invention into the spinal cord of the hemisection models 1 week after the hemisection. In the figure, "Mock" represents the negative control group that received HEPES buffer after SCI; "ChABC" is an enzyme that digests chondroitin sulfate glycosaminoglycans (chondroitinase ABC) having therapeutic effects on spinal cord injuries; "Cblnl" represents the full length of the human Cblnl protein; and "Sham" represents the group in which any spinal cord injury was not caused after myelotomy. In the figure, "w" denotes week(s). Note that unlike CPTX, Cblnl cannot form a complex of Nrx(S4+) and AMPA receptors due to the absence of a binding element to AMPA receptors.

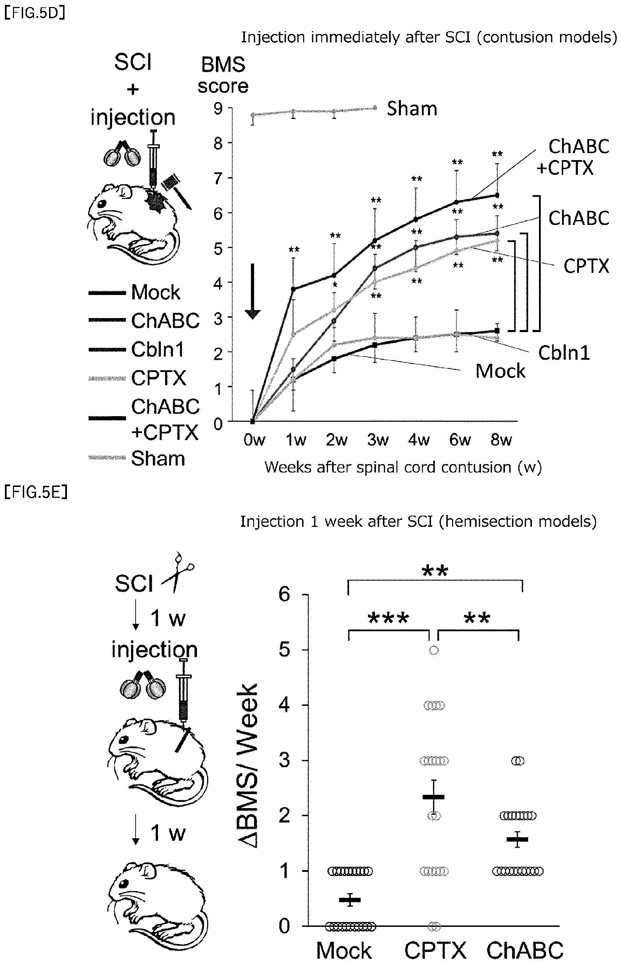

[Fig. 5D] Fig. 5D shows time-dependent changes in Basso Mouse Scale (BMS) scores after injecting a fusion protein of the present invention into the spinal cord of the contusion models immediately after injury. In the figure, "Mock" represents the negative control group that received HEPES buffer after SCI; "ChABC" is an enzyme that digests chondroitin sulfate glycosaminoglycans (chondroitinase ABC) having therapeutic effects on spinal cord injuries; "ChABC + CPTX" represents the group that received both of them simultaneously; "Cblnl" represents the full length of the human Cblnl protein; and "Sham" represents the group in which any spinal cord injury was not caused after myelotomy. In the figure, "w" denotes week(s). Note that unlike CPTX, Cbln 1 cannot form a complex of Nrx(S4+) and AMPA receptors due to the absence of a binding element to AMPA receptors.
[Fig. 5E] Fig. 5E shows changes in BMS scores (ΔBMS/week) of the Basso Mouse Scale (BMS) scores between the second and first week after injecting a fusion protein of the present invention into the spinal cord of the hemisection models 1 week after the hemisection. In the figure, "Mock" represents the negative control group that received HEPES buffer after SCI; and "ChABC" represents the group that received an enzyme that digests chondroitin sulfate glycosaminoglycans (chondroitinase ABC), which has therapeutic effects on spinal cord injuries.

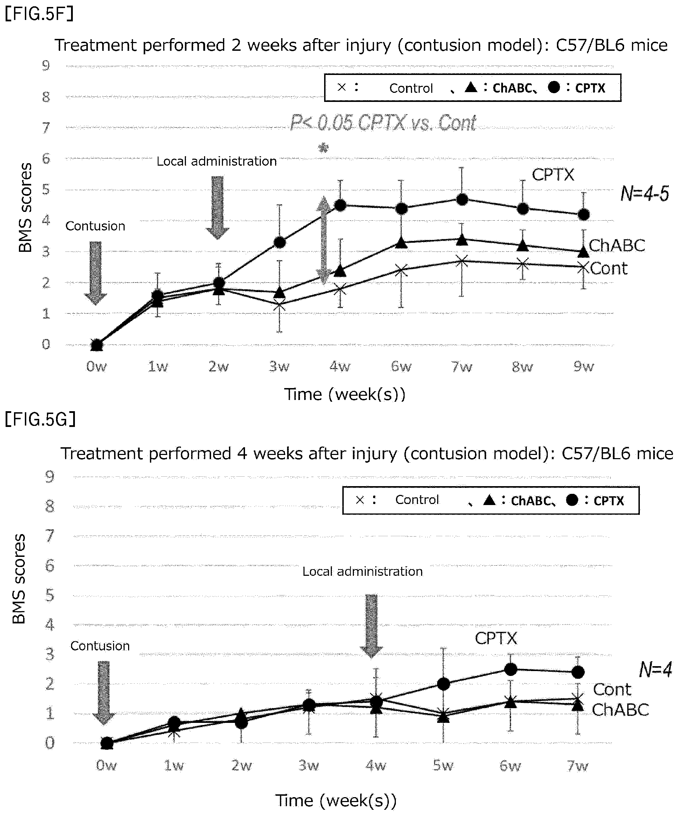

[Fig. 5F] Fig. 5F shows time-dependent changes in Basso Mouse Scale (BMS) scores after locally injecting a fusion protein of the present invention into the contusion models at the subacute phase (2 weeks after injury). In the figure, "Cont" represents the negative control that received HEPES buffer after SCI; and "ChABC" is an enzyme that digests chondroitin sulfate glycosaminoglycans (chondroitinase ABC) having therapeutic effects on spinal cord injuries. The contusion models were prepared at week 0 (0 w), and local injection was performed at 2 w. C57/BL6 mice were used as animal model.
[Fig. 5G] Fig. 5G shows time-dependent changes in Basso Mouse Scale (BMS) scores after locally injecting a fusion protein of the present invention into the contusion models at the chronic phase (4 weeks after injury). In the figure, "Cont" represents the negative control that received HEPES buffer after SCI; and "ChABC" is an enzyme that digests chondroitin sulfate glycosaminoglycans (chondroitinase ABC) having therapeutic effects on spinal cord injuries. The contusion models were prepared at week 0 (0 w), and local injection was performed at 4 w. C57/BL6 mice were used as animal model.

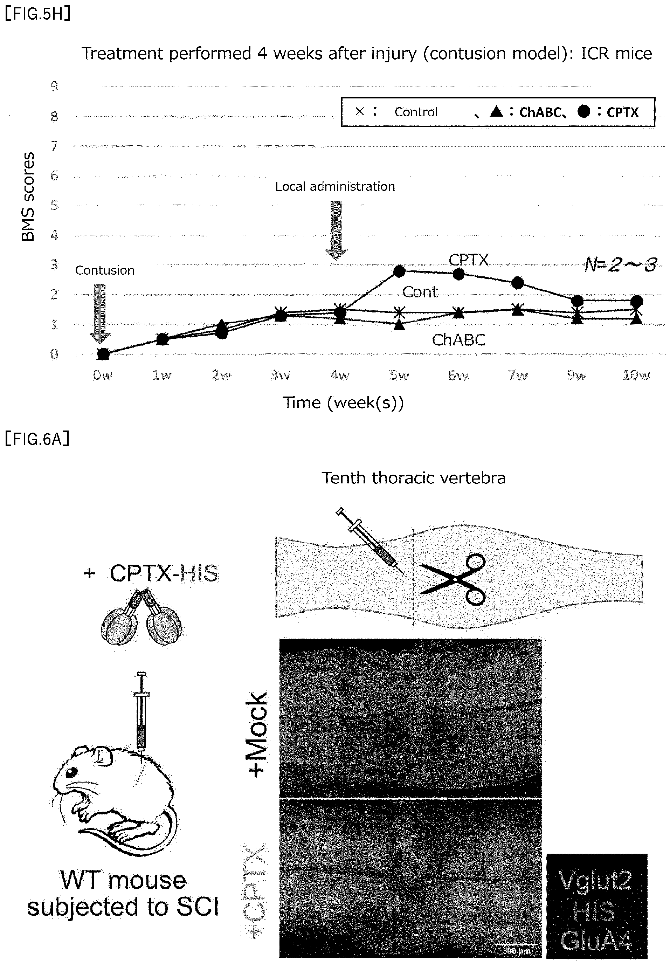

[Fig. 5H] Fig. 5H shows time-dependent changes in Basso Mouse Scale (BMS) scores after locally injecting a fusion protein of the present invention into the contusion models at the chronic phase (4 weeks after injury). In the figure, "Cont" represents the negative control that received HEPES buffer after SCI; and "ChABC" is an enzyme that digests chondroitin sulfate glycosaminoglycans (chondroitinase ABC) having therapeutic effects on spinal cord injuries. The contusion models were prepared at week 0 (0 w), and local injection was performed at 4 w. ICR mice were used as animal model.
[Fig. 6A] Fig. 6A shows fluorescence microscopy images indicating the co-localization of CPTX, Vglut2, and GluA4 after administering a fusion protein of the present invention (CPTX) to the spinal cord of the hemisection models. Fig. 6A shows that CPTX is concentrated around the sectioned site of the spinal cord.

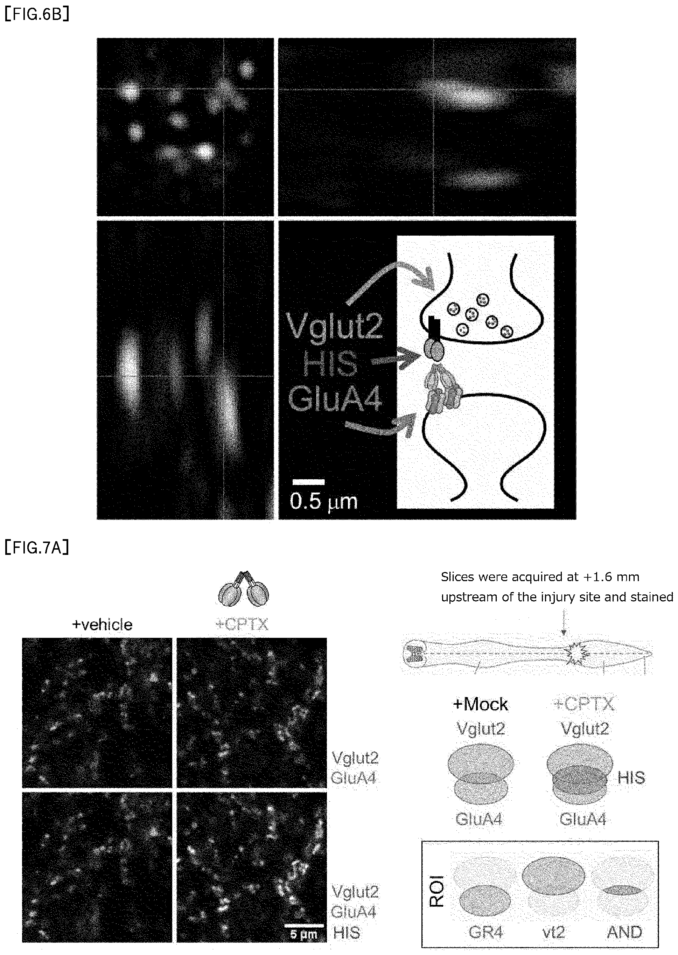

[Fig. 6B] Fig. 6B shows fluorescence microscopy images indicating the co-localization of CPTX, Vglut2, and GluA4 after administering a fusion protein of the present invention (CPTX) to the spinal cord of the hemisection models. Fig. 6B shows that CPTX is localized between Vglut2 and GluA4.
[Fig. 7A] Fig. 7A shows fluorescence microscopic images of tissue sections acquired at 1.6 mm upstream of the sectioned site in the hemisection models. Fig. 7A shows a region where the fluorescent signals for Vglut2 and GluA4 overlap. Fig. 7A also shows that CPTX is localized in the overlapping region.

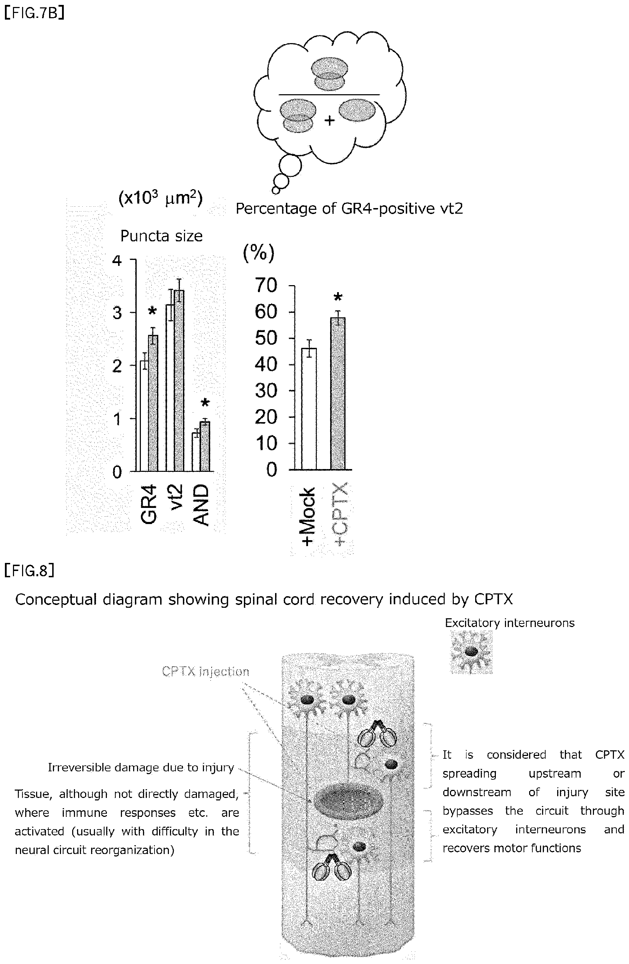

[Fig. 7B] Fig. 7B shows the effects of CPTX administration on the area of the fluorescent signal region for GluA4 (GR4) and Vglut2 (vt2), and the area of the region where they overlap (see Fig. 7B left). Fig. 7B also shows the effects of CPTX administration on the percentage of the GR4-positive vt2 region (see Fig. 7B right).
[Fig. 8] Fig. 8 is a schematic diagram of the anticipated process of recovery from a spinal cord injury, which has been achieved with a fusion protein of the present invention. In spinal cord injuries, the injury site itself may have irreversible damage. The area surrounding the injury site, although not directly damaged, is anticipated to become an environment where immune responses and other factors are activated, causing difficulty in the neural circuit reorganization. Conversely, the fusion proteins of the present invention can link excitatory intemeurons. Therefore, rather than directly repairing the injury site, the fusion proteins of the present invention are anticipated to induce neuronal circuit reorganization by linking the excitatory interneurons upstream or downstream of the injury site, thereby creating a neurotransmitter pathway that bypasses the spinal cord injury site and recovering from its damage.

### Detailed description of the invention

In the present invention, a "subject" refers to a vertebrate, e.g., a bird or mammal, e.g., a mammal such as a mouse, rat, hamster, guinea pig, horse, bovine, pig, goat, sheep, donkey, dog, and cat, and a primate such as a monkey, chimpanzee, gorilla, orangutan, bonobo, and human, and in particular, human. In this specification, the "subject" is used to include humans as described above, and the term "non-human" is used when humans are excluded.

As used herein, "treatment" means a medical intervention on a subject. The term "treatment" is used to include therapeutic treatment. The therapeutic treatment may provide therapeutic effects such as improving, inhibiting deterioration, reducing deterioration rate, cessation, and curing diseases, disorders, and conditions. In this specification, a "therapeutically effective amount" is the amount necessary for a therapeutic effect to exhibit.

In this specification, a "Cblnl protein" represents a protein that is also called precerebellin, a cerebellin 1 precursor, or cerebellin-1. Cblnl is a secreted protein belonging to the C1q family and is mainly produced in and secreted from the cerebellar granule cells. Cblnl binds to the presynaptic neurexin (Nrx). Cblnl is crucial for synapse formation between the parallel fibers and Purkinje cells (parallel fiber synapses). Additionally, the Cblnl knockout mice displayed reduced parallel-fiber synapse density and severe cerebellar ataxia symptoms. The human CBLN1 protein may have an amino acid sequence deposited as the NCBI Reference Sequence NP_004343.1. In the human CBLN1, the amino acid sequence at positions 1-21 is the signal sequence; the amino acids at positions 34-38 are reported to be essential for binding to NRXN1; cysteines at 34 and 38 are used to form a disulfide bond between CBLN1s; and the amino acids at 57-193 are the C1q domain. In the human CBLN1, the amino acids at 62-193 are reported to be necessary for binding to CBLN3 and homo-trimerization, and those at 122-147 are reported to be essential for interacting with GLUD2. The human CBLN1 contains a binding element for Nrx (Nrx-binding domain) in the amino acid region at 22-53. The Nrx-binding domain of the CBLN1 protein may have an amino acid sequence of the CBLN1 protein corresponding to the amino acid sequence at positions 22-53 of the human CBLN1 protein (NCBI Reference Sequence: NP_004343.1). The Nrx-binding domain may be responsible for binding to Nrx (i.e., Nrx(S4+)), which has splice site 4 (fourth splice site). Throughout this specification, gene and protein names, whether in the upper or lower case, are used to include orthologs of all mammalian species. In this specification, the animal species from which gene and protein names are derived are discriminated by describing the animal species in front of the gene or protein name.

In this specification, an amino acid sequence corresponding to a given amino acid sequence refers to an amino acid sequence situated at the matching location when aligned with the given amino acid sequence in orthologs and homologs including natural variants of the given amino acid sequence.

In this specification, "neuronal pentraxin-1," also called Nptxl or NP1, is a member of the neuronal pentraxin gene family. Nptxl binds to and activates AMPA-type glutamate receptors (AMPARs). The human NPTX1 protein may have an amino acid sequence deposited as the NCBI Reference Sequence: NP_002513.2. In this human NPTX1 protein, the amino acid sequence at positions 1-22 is the signal sequence, and the amino acid sequence at positions 222-428 is the pentraxin domain. The NPTX1 protein has a binding element for AMPAR (AMPAR binding domain) in the amino acid region at 222-428. The AMPAR-binding domain of the NPTX1 protein may have an amino acid sequence of the NPTX1 protein corresponding to the amino acid sequence at positions 222-428 of the aforementioned human NPTX1 protein (NCBI Reference Sequence: NP_002513.2). Herein, "AMPA" means alpha-amino-3-hydroxy-5-mesoxazole-4-propionic acid.

As used herein, the term "multimerization domain" means a domain that can multimerize a protein by linking it to the protein. A multimerization domain can be, for example, a coiled-coil domain. Examples of the multimerization domains include dimerization and trimerization domains. Trimerization domains are involved in protein trimerization in the natural proteins, and they can also trimerize the chimeric protein of the trimerization domain and the target protein. Therefore, the target protein can be trimerized by producing a chimeric protein with a trimerization domain. Trimerization domains include influenza hemagglutinin, SARS spike, HIV gp41, GCN4, modified GCN4, bacteriophage T4 fibrillin, and ATCase-derived trimerization domains. Also included in the trimerization domains are those of TRAF2, thrombospondin-1, Matrilin-4, and Matrilin-1. In this specification, a multimer may be a homo-multimer.

According to the present invention, a molecule that can bind to the antigens presented on the surface of the presynaptic and postsynaptic membranes and link them using the molecule has an effect of reconnecting pre- and post-synapses in the injury site after a spinal cord injury. According to the present invention, in particular, molecules that link Nrx and an AMPA-type glutamate receptor have an effect of reconnecting pre- and post-synapses in the injury site in a spinal cord injury. Thus, according to the present invention, fusion molecules of a Nrx-binding molecule and a AMPA-type glutamate receptor-binding molecule are provided. In an embodiment, the molecule can be a protein. Various molecules that bind to a given protein can be obtained by those skilled in the art, such as antibodies and fragments thereof with antigen specificity (e.g., monoclonal antibodies or fragments thereof, e.g., bispecific antibodies as well as fragments of bispecific antibodies such as bispecific single chain diabodies, bispecific tandem scFv, and bispecific F(ab)₂), aptamers (DNA aptamers or RNA aptamers {RNA aptamers may be those stabilized by modified nucleic acids}), and regions of the proteins given below. According to a preferred embodiment of the present invention, there is provided a multimer (e.g., a trimer, preferably a hexamer, in particular a hexamer consisting of two trimers linked to each other by a disulfide bond) of one or more fusion proteins (sometimes referred to as "fusion protein(s) of the present invention") in which each fusion protein contains a Nrx-binding region of a Cblnl protein, a multimerization domain, and a AMPA-type glutamate receptor-binding region of Nptx1.

According to the present invention, the Nrx-binding region of the Cblnl protein is capable of binding to Nrx containing splice site 4 (i.e., Nrx(S4+)). The Nrx-binding region of the Cblnl protein can be, for example, a region of Cbln1 corresponding to the cysteine-rich region (CRR) (J. Elegheert et al., Science353, 295-299 (2016)) of the human Cblnl (GenBank ID NM_004352; Gln22-Ile53) (a region of Cblnl having a corresponding amino acid sequence).

According to the present invention, any multimerization domain (or multimer-forming domain) can be used as long as it is capable of multimerizing fusion proteins. In an embodiment of the present invention, the multimerization domain can be a trimerization domain (or trimer-forming domain). The trimerization domain can be, for example, a coiled-coil domain (especially the one capable of forming a coiled-coil triple helix). For example, in a preferred embodiment, the trimerization domain can be, but not limited to, a GCN4 trimerization domain. Examples of the trimerization domain that can be used include those selected from the group consisting of trimerization domains of collagen family proteins (e.g., collagen α1 and α2), those of α-keratin, Clq protein, overwintering protein ACRP30, cerebellin, multimerin, collectin, conglutinin, pulmonary surfactant protein A (SP-A), and mannose binding proteins (MBPs). In a preferred embodiment, the trimerization domain of the Clq protein family and the collectin family can be used. The trimerization domain can have a collagen-like sequence.

According to the present invention, the AMPA-type glutamate receptor-binding region of Nptxl can be a pentraxin domain of the Nptxl protein. The AMPA-type glutamate receptor-binding region of Nptxl can be, for example, a region of Nptxl corresponding to a pentraxin domain of the human neuropentraxin-1 (NP1_{PTX}; GenBank ID AC50727.1; Pro224-Ile431) (a region of Nptxl having a corresponding amino acid sequence).

For the fusion proteins of the present invention, the multimerization domain in an embodiment may be interposed between the Nrx-binding region of the Cblnl protein and the AMPA-type glutamate receptor-binding region of Nptxl. This makes the fusion proteins of the present invention advantageous in mediating the binding of Nrx and AMPA-type glutamate receptors. The multimerization domain can also be sandwiched between the two regions to promote multimerization.

In an embodiment of the present invention, in the fusion protein of the present invention, the Nrx-binding region of the Cblnl protein, the multimerization domain, and the AMPA receptor-binding region of Nptxl may be connected via a linker. The linker can be a peptide. In addition, the linker can be, for example, a flexible linker. Examples of the flexible linkers include hydrocarbon linkers with -(CH₂)₆- or (GGGGS)ₙ (SEQ ID NO. 3), KESGSVSSEQLAQFRSLD (SEQ ID NO. 4) or EGKSSGSGSESKST (SEQ ID NO. 5), GGGGGGGG (SEQ ID NO. 6), GSAGSAAGSGEF (SEQ ID NO. 7), (GGSG)ₙ (SEQ ID NO. 8) or (GS)ₙ (SEQ ID NO. 9) {where n is a natural number from 1 to 5}. In an embodiment of the present invention, in the fusion proteins of the present invention, the Nrx-binding region of the Cblnl protein, the multimerization domain, and the AMPA receptor-binding region of Nptx1 may be directly connected without any linker.

In an embodiment of the present invention, regarding the fusion proteins of the present invention, the multimerization domain can be a trimerization domain, and they can be a trimer. Regarding the fusion proteins of the present invention, the multimerization domain can be a trimerization domain, and they can be a hexamer consisting of two trimers linked to each other. Two trimers can be linked to form a hexamer through the cysteine residues 34 and 38 of the human Cblnl protein (NCBI Reference Sequence: NP_004343.1).

In an embodiment of the present invention, any of the regions and domains of the fusion proteins of the present invention can be derived from human proteins. In an embodiment of the present invention, a fusion protein of the present invention can form a multimer of one or more fusion proteins, each fusion protein containing a Nrx-binding region of the human Cblnl protein, a multimerization domain of a human protein, and a AMPA receptor-binding region of human Nptxl, the multimerization domain being a trimerization domain, and the fusion protein can be in a trimeric form or a hexameric form.

In an embodiment of the present invention, the fusion protein of the present invention can have an amino acid sequence of SEQ ID NO. 2. In an embodiment of the present invention, a nucleic acid encoding a fusion protein of the present invention can have a nucleotide sequence of SEQ ID NO. 1 (or a corresponding mRNA sequence).

The fusion proteins or multimers thereof according to the present invention can bridge Nrx proteins expressed in the presynaptic terminals of the presynaptic cells and AMPARs expressed in the dendrites of the postsynaptic cells (see Fig. 1B), thereby forming new connections between the excitatory interneurons in the spinal cord. Thus, the fusion proteins of the present invention can be used to form new connections (e.g., synaptic connections) between the excitatory interneurons (e.g., between the excitatory interneurons in the spinal cord). According to the present invention, such new connections can be formed around or in the vicinity of spinal cord injuries. The term "around" can refer to, for example, within 0.5 cm, 1 cm, 2 cm, 3 cm, 4 cm, 5 cm, 6 cm, 7 cm, 8 cm, 9 cm, 10 cm, 20 cm, 30 cm, 40 cm, or 50 cm from the injury site. The term "in the vicinity" can refer to, for example, within 0.5 cm, 1 cm, 2 cm, 3 cm, 4 cm, 5 cm, 6 cm, 7 cm, 8 cm, 9 cm, or 10 cm from the injury site.

The fusion proteins or multimers thereof according to the present invention can bridge Nrx(S4+) in the presynaptic cells and AMPARs in the postsynaptic cells via themselves. Thus, the fusion proteins or multimers thereof according to the present invention can be used to link Nrx(S4+) in the presynaptic cells and AMPARs in the postsynaptic cells. Promotion of the connection between Nrx(S4+) in the presynaptic cells and AMPARs in the postsynaptic cells can promote the connection between the excitatory interneurons. Thus, the fusion proteins or multimers thereof according to the present invention can be used to promote the connection between the excitatory interneurons.

According to the present invention, pharmaceutical compositions including one or more fusion proteins or a multimer thereof according to the present invention (sometimes referred to as "pharmaceutical composition(s) of the present invention") are provided. The pharmaceutical compositions of the present invention may contain a pharmaceutically acceptable excipient. Examples of the excipients include aqueous solvents, buffers, surfactants, antimicrobial agents, and bulking agents. In an embodiment, an aqueous solvent may be water for injection, saline, or artificial cerebrospinal fluid. The pharmaceutical compositions of the present invention may be lyophilized and can be provided as lyophilized formulations (or may be provided as a kit with a lyophilized formulation and, if desired, an aqueous solvent for use preparation as needed).

The pharmaceutical compositions of the present invention can be used to treat spinal cord injuries. The pharmaceutical compositions of the present invention can also be used to promote the connection between neurons (especially between excitatory interneurons) in a region surrounding a spinal cord injury site.

The pharmaceutical compositions of the present invention can be administered intraspinally. The pharmaceutical compositions of the present invention can be administered, for example, at an upstream site in the vicinity of a spinal cord injury to improve motor function and/or sensory function. As used herein, the term "upstream" means the direction from the injury site toward the central nervous system. Improvement in the motor function and/or sensory function can be tested using a method known to those skilled in the art. For example, improvement in the function can be assessed using a neurological assessment method such as the American Spinal Injury Association (ASIA) motor score, the ASIA impairment scale (AIS), and the Frankel classification.

In an embodiment of the present invention, the subject is the one with a spinal cord injury, e.g., a subject with an acute spinal cord injury, a subject with a subacute spinal cord injury and/or a subject with a chronic spinal cord injury. For example, the subject is a human with a spinal cord injury, e.g., a human with an acute spinal cord injury, a human with a subacute spinal cord injury and/or a human with a chronic spinal cord injury. The acute phase refers to the period of pronounced biological and biochemical responses that follow a primary injury caused by external forces on the spinal cord. During secondary injuries, cell death of nerve and glial cells is caused due to, for example, hematoma, ischemia, edema, and cytotoxicity resulting from inflammatory cell infiltration and neurotransmitter leakage. The subacute phase refers to the period when the inflammation during the acute phase has subsided and angiogenesis and tissue repairs occur actively. The chronic phase refers to the period when cavities are formed and tissue loss occurs at the site of spinal cord injury, and astrocytes form a rigid glial scar around the injury site, forming a barrier to axonal regeneration. The subject can be within 1, 2, 3, 4, 5, 6, 7, 8, 9, 10, 14, 21, or 28 days, or 2, 3, 4, 5, or 6 months, or 1, 2, 3, 4, or 5 years, or more, after having suffered a spinal cord injury.

It is known that in normal human spinal cord injuries, some nerves remain even after severe injuries. Thus, in an embodiment of the present invention, the subject may have a partial damage to the spinal cord. A partial damage refers to an injury with some connections between upstream and downstream of the injury site of the spinal cord are maintained. Nerve connections can be determined using anatomical examination and/or electrophysiological examination. In an embodiment of the present invention, the subject can have a complete spinal cord injury (e.g., complete disconnection). If the entire spinal cord has damaged, for example, a pharmaceutical composition of the present invention may be administered along with a procedure to join the injured surfaces together.

The fusion proteins of the present invention can be those of proteins derived from the same species as the animal to be administered.

According to the present invention, nucleic acids encoding a fusion protein of the present invention are provided. The nucleic acids include DNA and RNA.

The pharmaceutical compositions of the present invention may contain, instead of the fusion protein of the present invention, mRNA encoding the fusion protein of the present invention and/or a gene expression vector comprising a nucleic acid encoding the fusion protein of the present invention operably linked to a promoter.

The fusion protein of the present invention can be produced from a nucleic acid encoding the fusion protein. The fusion protein of the present invention can be obtained by expressing a nucleic acid encoding the fusion protein of the present invention as mRNA in cells (e.g., mammalian cells such as human cells, E. *coli,* or insect cells). According to the present invention, gene expression vectors comprising a nucleic acid encoding the fusion protein of the present invention (gene expression vectors of the present invention) operably linked to a promoter are provided. The gene expression vectors that can be used include plasmid vectors and epizomal vectors such as Sendai virus vectors, as well as vectors inserted into nuclear genomes such as retrovirus vectors. Gene expression vectors of the present invention can comprise an element selected from the group consisting of replication origin, promoter, nucleic acid encoding a fusion protein of the present invention operably linked to the promoter sequence, and drug selection markers. As the promoter, those enabling transcription of mRNA encoding a fusion protein of the present invention in cells (e.g., mammalian cells such as human cells, E. *coli,* or insect cells) can be used, and those skilled in the art can appropriately select one from known promoters (e.g., CMV promoters, RSV promoters, MMT promoters, metallothionein promoters, heat shock promoters, albumin promoters, ApoA 1 promoters, human globin promoters, retrovirus LTRs, human growth hormone promoters, beta-actin promoters, adenovirus promoters, thymidine kinase promoters, and B19 parvovirus promoters). Drug selection markers may be used or not, but if any, only cells carrying a gene expression vector can be allowed to survive by treating the cells with the drug.

According to the present invention, recombinant cells (e.g., mammalian cells such as human cells, E. *coli,* or insect cells) having a nucleic acid encoding the fusion protein of the present invention are provided. Examples of the cells include Chinese hamster ovary cells (CHO cells) and 293T cells. The 293T cells may be cultured, for example, in the presence of the class I alpha-mannosidase inhibitor kifunensine to express the fusion protein of the present invention. The cells used in which the fusion protein of the present invention is expressed may be GnTI-/- cells (homozygous for the knocked-out GnTI gene), such as HEK293S-GnTI-/- (e.g., ATCC (trademark) CRL-3022 TM). The media and culture conditions can be suitable for protein expression and can be determined by a person skilled in the art.

The nucleic acids encoding the fusion protein of the present invention can have a signal sequence. The fusion proteins or multimers thereof according to the present invention may or may not have a signal sequence.

According to the present invention, multimers of one or more fusion proteins of the present invention are secreted as multimers from cells. In particular, fusion proteins of the present invention with a trimerization domain as the multimerization domain can be secreted as a hexamer from cells. In other words, the multimers of the one or more fusion proteins can be obtained by culturing cells capable of expressing the one or more fusion proteins under conditions suitable for the formation of multimers. Under these conditions, a hexamer can be formed spontaneously by self-assembly of monomers in the culture medium. Thus, the fusion protein of the present invention can be collected in the form of a multimer (e.g., hexamer), from a culture supernatant of recombinant cells. The resulting multimer can be purified by a technique known to those skilled in the art. The purified multimer can be formulated with its multimeric structure kept unchanged.

According to the present invention, the fusion proteins of the present invention may be incubated under conditions suitable for multimerization, if necessary, or under conditions suitable for forming a disulfide bond between the fusion proteins. According to the present invention, the fusion proteins of the present invention may be stored under conditions of a solution suitable for maintaining the multimeric state. The present invention provides a method for producing a hexamer of the one or more fusion proteins of the present invention, the method including:
culturing cells having one or more nucleic acids having a signal sequence and encoding one or more fusion proteins of the present invention under a condition suitable for protein expression;
acquiring a culture supernatant; and
collecting a hexamer of one or more fusion proteins from the acquired culture supernatant.
Moreover, the present invention also provides a method for producing a fusion protein of the present invention, the method including culturing 293T cells having a nucleic acid encoding a fusion protein of the present invention in the presence of the class I alpha-mannosidase inhibitor kifunensine, and collecting a hexamer of the fusion protein of the present invention thus expressed, from a cell culture supernatant. The present invention further provides a method for producing a fusion protein of the present invention, including culturing GnTI-/- cells (e.g., human GnTI-/- cells, such as HEK293S-GnTI-/- cells) having a nucleic acid encoding a fusion protein of the present invention under conditions suitable for protein expression, and collecting a hexamer of the fusion protein of the present invention thus expressed, from a cell culture supernatant. The aforementioned methods of the present invention may further include purifying the collected hexamer. The aforementioned methods of the present invention may further include mixing the purified hexamer with a pharmaceutically acceptable excipient to produce a pharmaceutical composition. The hexamer of the fusion protein(s) of the present invention may be stored under conditions suitable for maintaining the disulfide bond linking the two trimers, such as non-reducing conditions, because reducing conditions may cleave the disulfide bond linking the two trimers. The methods of the present invention may further include determining how many monomer units of the fusion protein(s) are linked before collecting the multimer of the fusion protein(s). The hexameric nature for the multimer of the fusion protein(s) can be confirmed using, for example, a multi-angle light scattering detector.

According to the present invention, there is provided the use of a multimer of the fusion protein(s) of the present invention in forming new synaptic connections between excitatory interneurons.

According to the present invention, there is provided the use of a multimer of the fusion protein(s) of the present invention in manufacturing a medicament for use in forming new synaptic connections between excitatory interneurons.

According to the present invention, there is provided the use of a fusion protein of the present invention in manufacturing a medicament for use in forming new synaptic connections between excitatory interneurons.

According to the present invention, there is provided the use of a nucleic acid encoding a fusion protein of the present invention in manufacturing a medicament for use in forming new synaptic connections between excitatory interneurons.

According to the present invention, there is provided the use of recombinant cells having a nucleic acid encoding a fusion protein of the present invention in manufacturing a medicament for use in forming new synaptic connections between excitatory interneurons.

According to the present invention, trimers of one or more fusion proteins of the present invention are provided. The trimers of the fusion protein(s) of the present invention have a trimerization domain in each of the fusion protein(s), and form a trimeric form by the trimerization domains. According to the present invention, hexamers of the fusion protein(s) of the present invention are provided. The hexamer of the fusion protein(s) of the present invention consists of two trimers of the fusion protein(s) of the present invention linked to each other by a disulfide bond. The hexamer of the fusion protein(s) of the present invention has either or both sulfhydryl groups of cysteine residues corresponding to the cysteine residues 34 and 38 of the amino acid sequence deposited as the NCBI Reference Sequence NP_004343.1, and the two trimers are linked through the formation of a disulfide bond between the sulfhydryl groups. The hexamers of the fusion protein(s) of the present invention can be obtained as a hexamer of fusion protein(s) of the present invention without any signal sequence, when the fusion protein(s) of the present invention is/are expressed in cells having nucleic acid(s) encoding the fusion protein(s) of the present invention with a signal sequence by the method described above. Thus, the hexamers of the fusion protein(s) of the present invention can be provided without any signal sequence. According to the present invention, pharmaceutical compositions containing a hexamer of the fusion protein(s) of the present invention are provided. The pharmaceutical compositions may further contain a pharmaceutically acceptable excipient. The hexamers of the fusion protein(s) of the present invention are maintained in conditions of a solution in which the disulfide bond linking the two trimeric units is stable.

The present invention provides a method of treating a subject, including administering to the subject a therapeutically effective amount of a multimer of one or more fusion proteins of the present invention. The subject may have a spinal cord injury.

The present invention provides a method of treating a subject, including administering to the subject a therapeutically effective amount of a nucleic acid encoding a fusion protein of the present invention. The subject may have a spinal cord injury.

The present invention provides a method of treating a subject, including administering to the subject a therapeutically effective amount of cells (which belong to the same species as but are a different strain from the subject or belong to a different species from the subject) containing a nucleic acid encoding a fusion protein of the present invention. The subject may have a spinal cord injury.

According to the present invention, the subject can be treated with a multimer of the fusion protein(s) of the present invention in combination with other therapeutic agent(s) for spinal cord injuries. According to the present invention, the subject can also be subjected to rehabilitation in addition to the treatment method according to the present invention.

### Examples

### Materials and Methods

### Mice

All behavioral experiments were performed in the late afternoons during the dark phase cycle when mice were active, under constant temperature (22°C ± 1°C) and humidity (50% ± 10%). The spinal cord injury experiments were performed following the animal experimental regulations of Aichi Medical University (approval numbers: 1559, 2019-89, 1642, 2020-48).

### Plasmids

For surface plasmon resonance (SPR) assays, cDNAs encoding the extracellular human glutamate receptor D2 amino-terminal domain ATD (GenBank ID NM_001510; GluD2 ATD: Asp24-Gly440), the human glutamate receptor A4 ATD (GenBank ID U16129.1; GluA4 ATD: Gly21-Thr416), the human betaneurexin-1 LNS6 domain (GenBank NM_NM_138735; beta-Nrx LNS6: His85-D265), and the pentraxin domain of human neuropentraxin-1 (NP1_{PTX}; GenBank ID AC50727.1; Pro224-Ile431) were fused to the C-terminus of a hexa-histidine (His6) tag and cloned into the pHLsec expression vector (A. R. Aricescu et al., Acta Crystallogr D Biol Crystallogr 62, 1243-1250 (2006)). Then, to produce a trimeric form of the pentraxin domain, NP1_{PTX} was fused to the C-terminus of a three-stranded GCN4 leucine zipper coiled-coil sequence (P. B. Harbury et al., Science 262, 1401-1407 (1993)), thereby yielding NP1_{PTX-3COIL}. Then, to produce CPTX, NP1_{PTX-3COIL} was fused to the C-terminus of the cysteine-rich region (CRR) (J. Elegheert et al., Science 353, 295-299 (2016)) of the human Cblnl (GenBank ID NM_004352; Gln22-Ile53) (see Fig. 1B). For the *in vitro* binding and synapse formation assays, the full-length mouse NP1 (GenBank ID NM_008730.2) was cloned into the pCAGGS vector (kindly provided by Dr. J. Miyazaki, Osaka University) with a C-terminal HA-tag; neurexin full-length cDNA was cloned into the pCAGGS vector with a C-terminal FLAG-tag; a neurexin extracellular domain was cloned into the pCAGGGS vector with a C-terminal human Fc tag; and ATD(s) of any one of GluA1-4 were cloned into a modified pDisplay vector (Invitrogen), as previously described (K. Matsuda et al., Neuron 90, 752-767 (2016)). The miR sequences are inserted upstream of the IRES-EGFP sequence in the pCAGGS vector as previously described (Y. H. Takeo et al., J Neurosci 35, 12518-12534 (2015)). The 21-bp target sequences of the mouse Nptxl and Nptxr genes were designed by BLOCK-iT (Invitrogen) as: AGA CAA GTT TCA GCT GAC ATT (for NP1, SEQ ID NO. 10) and TGC TCA GTC GCT TCT TCT GTA (for NPR, SEQ ID NO. 11). For the immunocytochemical analyses to check the selectivity of anti-NPs antibodies, full-length mouse NP1 (GenBank ID NM_008730.2), NP2 (GenBank ID NM_016789.3), and NPR (GenBank ID NM_030689.4) were cloned into the pCAGGS vector with a C-terminal HA-tag. Additionally, for the immunoblotting analyses to check the selectivity of anti-NP antibodies, mouse NP1 (without the signal sequence: aa1-22), NP2 (without signal sequence: aa1-14), and NPR (without the transmembrane domain: aa1-23) were cloned into the pCAGGS with the Igκ signal sequence and a successive N-terminal 2xHAtag.

### Preparation of the recombinant proteins

Proteins were expressed using transient transfection of the HEK293T cells for large-scale protein production. Five days after transfection, the conditioned medium was collected and buffer exchanged using a QuixStand benchtop diafiltration system (GE Healthcare). It can be considered that the recombinant proteins should be secreted into the culture supernatant and present in a hexameric form with two trimers linked to each other (see Fig. 2). The recombinant proteins were purified by immobilized metal-affinity chromatography (IMAC) using pre-packed nickel Sepharose columns (GE Healthcare). The proteins were concentrated and further purified using size exclusion chromatography (SEC; Supferdex 200 16/60 PG HiLoad column; GE Healthcare) in 10 mM HEPES (4-(2-hydroxyethyl)-1-piperazine ethane sulfonic acid) pH 7.40, 150 mM sodium chloride, and 3 mM calcium chloride (HBS-C) for structural studies, or in 10 mM Tris pH 7.4, 150 mM sodium chloride, 3 mM calcium chloride, and 0.005% (v/v) Tween-20 (TBS-CT) for interaction studies using SPR. Note that Cblnl is considered to form a trimer without a disulfide bond responsible for its multimerization; however, hexamers were largely secreted since trimers are not easily secreted out of the cells.

### Multi-angle light scattering (MALS)

Concentrated protein samples of approximately 1.0 g/L were injected into an HPLC-driven SEC column (Superdex 200 10/30 column, GE Healthcare) equilibrated with HBS-C buffer. The SEC column was coupled to an online UV detector (Shimadzu), an 18-angle light scattering detector (DAWN HELEOS), and a refractive index detector (Optilab T-rEX) (Wyatt Technology). The MALS proteins contained an N-linked oligo-mannose sugar, and their molecular weights were determined using an adapted RI increment value (dn/dc standard value; 0.185 mL/g) to account for the glycosylation state. Data analysis was performed using the ASTRA V software (Wyatt Technology). According to the multi-angle light scattering, the resulting recombinant proteins had a molecular weight of 208.9 ± 3.3 kDa (see Fig. 3); thus, it was considered that hexamers were formed as expected.

### Antibodies

The origins, dilutions, companies and catalog numbers are as follows: anti-calbindin (goat, 1:500, Frontier Science, Af1040); anti-HIS (mouse, 1:1000, MBL, D291-3 or rabbit, 1:1000, CST, #2365); anti-Myc (rabbit, 1:1000, MBL, 562); anti-FLAG (rabbit, 1:1000, SIGMA, F7425); anti-HA (mouse, 1:1000, BAbCo, MMS-101); anti-synaptophysin (mouse, 1:500, SIGMA, S5768 or G, 1:500, Frontier Science, Af300); antineurexin (chicken, 1:500, a gift from Dr. Peter Scheiffele (C. Dean et al., Nat Neurosci 6, 708-716 (2003)); anti-GFP (rabbit 1:1000, Frontier Science, Af-2020); anti-GluA1 (rabbit, 1:100, Calbiochem, PC246 or guinea pig, 1:500, Frontier Science, AF380); anti-GluA2/3 (rabbit, 1:1000, Chemicon, AB1506); anti-GluA4 (rabbit, 1:1000, Pharmingen, 60666N or guinea pig, 1:500, Frontier Science, Af640); anti-VGluT1 (rabbit, 1:500, Frontier Science, Af570 or goat, 1:500, Frontier Science, Af310); anti-VGAT (goat, 1:500, Frontier Science, Af620); anti-Parvalbumin (goat, 1:500, Frontier Science, Af460); anti-Homer 1 (guinea pig, 1:1000, Synaptic Systems, 160-004); and anti-Bassoon (rabbit, 1:500, Synaptic Systems, 141-003). Guinea pigs immunized with peptides were used to freshly raise the anti-NPl and NPR (NP1: aa 185-227 and NPR: aa 226-263). The mixture of the aforementioned primary antibodies was used to simultaneously detect the various GluAs (i.e., GluA1-3 or GluA1-4). Secondary antibodies conjugated with DyLight 405, Alexa 488, 546, 647 and Cy3 (Invitrogen or Jackson Lab) against the respective primary antibodies were used for dilution (1:1000 for immunocytochemistry or 1:200 for immunohistochemistry).

Cells and conditioned medium were solubilized in Laemmli buffer (2% SDS, 80 mM Tris-HCl pH 6.8, 10% glycerol, 0.00625% Coomassie blue G250) and boiled for 3 minutes using 2% 2-mercaptoethanol to reduce the proteins. Samples were subjected to SDS-PAGE in a gradient gel (Wako) and blotted onto a membrane (Millipore). The membrane was blocked with TS-tween (0.1% Tween-20, 50 mM Tris-HCl pH 7.6, 150 mM NaCl) containing 5% skim milk (Meiji) and incubated with the primary antibody for 2 hours and HRP-conjugated secondary antibody (GE Healthcare) for 30 minutes. Chemiluminescence was generated by an ImmunoStar detection kit (Wako) or Immobilon (Millipore) and detected using the LAS-3000 mini-system (FUJI FILM).

HEK293 cells were cultured in a high glucose DMEM (SIGMA) containing 10% FBS (HyClone), 50 U/mL penicillin, 50 mg/mL streptomycin (Invitrogen), and 2 mM L-glutamine at 37°C. The cells were transfected with the pDisplay encoding myc-tagged GluA1-GluA4-ATD or with the pCAGGS encoding FLAG-tagged Nrx or GFP using Lipofectamin 2000 (Invitrogen). The next day, the transfected cells were detached using PBS containing 5 mM EDTA and seeded on the 12-mm coverslips coated with PLL at 2 × 10⁴ cells/well. One hour after seeding, they were treated with the vehicle, recombinant Cblnl-HIS or CPTX-HIS (23.6 nM, final concentration as hexamers) for from 4 hours to overnight. For the *in vitro* tripartite binding assay, after an initial 4h ligand treatment and a single wash with a fresh culture medium, the cells were treated for 4 hours with a conditioned medium containing Nrx1β(+4)-hFc. The cells were fixed with 4% PFA/PBS for 15 minutes and washed thrice with PBS. After blocking using 3% BSA/PBS for 30 minutes without permeabilization, the cells were incubated with primary antibodies against the tags (HIS, HA, hFc) in the ligands in 3% BSA/PBS for 2 hours at room temperature or 24 hours at 4°C. After washing with PBS three times, the cells were permeabilized with PBS containing 0.1% TX 100 and 3% BSA. Then, the cells were stained with primary antibodies against tags (FLAG, Myc) in the receptors for 2 hours at room temperature or 24 hours at 4°C. Afterward, they were washed with PBS and stained with the respective secondary antibodies for 30 minutes. After washing with PBS, coverslips were mounted on a glass slide with Fluoromount-G. Samples where the HEK cells expressed GFP, were not stained with the primary antibody against GFP but treated with the same primary antibody against the receptor tag (FLAG or Myc). The auto-fluorescence of the GFP protein was detected using microscopy.

### Immunocytochemistry and Immunohistochemistry

Spinal cord sections. Mice were transcardially perfused with 3% glyoxal for 10 minutes under deep pentobarbital anesthesia 2-5 days after the injection. Next, dissected spinal cords were fixed with 3% glyoxal overnight at 4°C, cryoprotected in 30% sucrose/PBS for several days, and sliced. A cryostat (Leica) at 20-40 µm thickness in 4-5 mm around the injury's epicenter was used to horizontally or coronally section the spinal cords embedded in the Tissue-Tek O.C.T. compound (Sakura Finetek, #4583). The slices were mounted on APS-coated glass slides (Matsunami) at 200-400 µm intervals. After washing with PBS containing 0.1% TX-100, the sections were treated with 10% donkey serum for 30 minutes at room temperature and then with a mixture of primary antibodies and a mixture of respective secondary antibodies overnight. Finally, the sections were attached to the glass slides and mounted with Fluoromount-G (Southern Biotech). Fluorescence images of all sections on the glass slides were captured as a virtual slide using a conventional fluorescence microscope (BX63, Olympus) with a 4× objective to determine sections at 1.4-1.6 mm upstream of the injury's exact epicenter. Afterwards, the fluorescence images of the sections were captured using a confocal microscope (SD-OSR, Olympus; 63× objective) around the ventral root in the gray matter using the same parameters such as laser power, exposure time, gain, and offset for comparing samples. Airyscan 2 with LSM980 (63×/1.40 oil objective with 2.5× digital zoom, 35 nm/px, XY < 120 nm, Z < 350 nm) gave the super-resolution images. To define the particles of GluA4, VGluT2, and their intersections, the fluorescence images were subjected to background subtraction (50 px), Laplace filtering (9 × 9), box filtering (5 × 5), auto thresholding (Otsu method), particle extraction (> 5 px), binarization, and segmentation by watershed. The defined particles were used as ROI in quantifying each channel's size and mean intensity. The percentage of VGluT2 puncta with GluA4 was calculated by dividing the number of VGluT2 particles with more than 1 px of the defined GluA4 particles in the ROI by the total number of VGluT2 particles. All image processing procedures were performed using ImageJ.

Intraspinal injection of the CPTX injection solution. CPTX, Cblnl, chondroitinase ABC (Sigma Aldrich, C2905), or excipient (control) was injected in the proximity of the spinal cord injury site. An electrical microinjector (BJ-110, BEX CO., LTD.) through glass capillaries (3-000203-G/X, Drummond Scientific Company) at a concentration of 0.5 µl (CPTX, Cblnl, solvent; 1 µg/µl) or 0.5 µg (chondroitinase ABC; U/µl) was used for the injections. The muscle layers and skin were sutured to close after the respective injections.

### Spinal cord injury model

Mice (9-11 weeks old, ICR) were subjected to a compression-induced (see a contusion model in Fig. 4) or a hemisection-induced spinal cord injury (SCI; see a hemisection in Fig. 4). After anesthesia treatment, the spinal cords were surgically exposed, and the dorsal column at the tenth thoracic vertebra was dissected using micro-scissors or a scalpel for unilateral incision. Alternatively, mice were subj ected to a compression-induced injury using a commercially available SCI impactor device (Infinite Horizon Impactor; Precision Systems and Instrumentation, Lexington, NY) (K. Takeuchi et al., Nat Commun 4, 2740 (2013)). A 70-kdyn impact force was used. This device reports data in time versus force and time versus displacement. Then, 2 µL (1 µL × 2 sites) of 1.6 mg/mL CPTX (i.e., 15 pmol) or 1 µL (0.25 for four different sites) were administered near the injury site immediately after SCI or 1, 2, or 4 weeks later (the recovery effect for each dose was comparable). The other groups were also administered at a dose of 15 pmol each. The muscle layers and skin were sutured to close. The animals were recovered from the anesthetic after the administration of an antagonist.

Recovery of behavior and spontaneous locomotion in SCI-induced animals was assessed using the video recordings in a previous report (K. Takeuchi et al., Nat Commun 4, 2740 (2013)). The Basso Mouse Scale (BMS) open-field scoring (D. M. Basso et al., J Neurotrauma 23, 635-659 (2006)) and footfall tests were performed weekly. Two or more investigators blinded to the test groups assessed functional recovery during the 6-8 weeks after SCI. Mice with an incomplete injury (BMS score > 0 on that day) on day 3 after the SCI induction were excluded. The difference in the BMS scores between the first and second week after administration was calculated as ΔBMS/week (Fig. 5E). For each footfall test, mice were placed on a wire-mesh grid and videotaped for 5 minutes while on the grid. The mice that walked at least 70 times on the grid for 3 minutes were subjected to analysis, and the number of footfalls from the grid was counted as a footfall number (Fig. 5B).

### Results

The constructed artificial excitatory synaptic connector (named CPTX, a chimeric protein consisting of a Nrx-binding domain of Cbln1, a trimerization domain, and an AMPA receptor-binding domain of Nptx1) artificially bridges the AMPA receptor (AMPAR) and Nrx, thus stimulating the AMPAR and activating its downstream signaling (Fig. 1B).

This chimeric protein formed a hexamer, as expected, and was collected from the cell culture supernatant (Figs. 2 and 3).

As the spinal cord injury models, hemisection models were prepared by performing spinal cord hemisections with scissors and administered with the aforementioned artificial excitatory synaptic connector immediately after injury. Consequently, as shown in Figs. 5A and 5B, CPTX induced statistically significant and enhanced recovery (improvement in BMS scores) 1-2 weeks after the administration. Moreover, as shown in Figs. 5C and 5E, the administration 1 week after injury also induced significant recovery. Therefore, this effect is worthy of special mention considering that no treatment method has previously shown a prominent effect when administered 1 week after injury. Furthermore, even when the spinal cord was entirely dissected with a knife as a spinal cord injury model, CPTX showed a recovery effect to some extent when the severed surfaces were kept in contact.

Next, as the spinal cord injury models, contusion models were prepared and administered with the artificial excitatory synaptic connector CPTX immediately after injury. Consequently, as shown in Fig. 5D, CPTX induced statistically significant and enhanced recovery (improvement in BMS scores) 1 week after the administration. The combined use of CPTX and ChABC had a stronger effect. This result indicates that CPTX and the conventional ChABC agent have different mechanisms of action.

Next, CPTX was administered to the contusion models at the subacute (2 weeks after injury) and chronic (4 weeks after injury) phases, and its effects were evaluated. Contusion models (C57/BL6 mice, n = 4-5) were made using an impactor (70 kdyn, 1.5 mmΦ) by compressing their spinal cord, and 1 µL total of 1.7 mg/mL CPTX solution was locally administered in four doses of 0.25 µL over 1 minute in the vicinity of the injured area to spread over the injured area 2 weeks after injury. Consequently, as shown in Fig. 5F, CPTX induced highly prominent recovery, starting 1 week after the local administration. Additionally, a stronger contusion models (C57/BL6 mice, n = 4) were prepared (90 kdyn, 1.5 mmΦ impactors) and 1 µL total of 1.7 mg/mL CPTX solution was locally administered at a rate of 1 µL per minute to the injured area 4 weeks after injury. Consequently, as shown in Fig. 5G, CPTX induced a strong recovery tendency, starting 1 week after the local administration. A similar experiment to the one in Fig. 5G was performed by changing the mice to ICR mice (n = 2-3). Consequently, as shown in Fig. 5H, CPTX induced highly prominent recovery, starting 1 week after the local administration. Therefore, CPTX displayed obvious effects during the acute phase after the injury, and when administered during the subacute and chronic phases.

Next, His-tagged CPTX was administered to the SCI's injury site; subsequent expression and localization of Vglut2, His, and GluA4 (a subunit of AMPAR) were observed via immunohistochemistry. The administered CPTX accumulated at the injury site, and the expression of Vglut2 and GluA4 was observed in the surrounding area (see Fig. 6A). The long-resolution image analysis showed that signals derived from Vglut2 and GluA4 were observed above and below His (i.e., CPTX), respectively, demonstrating that CPTX bridges Vglut2 and GluA4, as shown in Fig. 6B. Notably, Vglut2 was used to observe the presynaptic site at excitatory synapses.

Tissue sections at 1.6 mm upstream of the sectioned site in the hemisection models were also observed 1 week after dissection (see Fig. 7A, upper right). In the tissue at 1.6 mm upstream, signals derived from Vglut2 and GluA4 were observed above and below His (i.e., CPTX), respectively, demonstrating that CPTX bridges Vglut2 and GluA4. The percentage of Vglut2 and GluA4 (GR4) co-localization in the tissue was determined. Specifically, the percentage of GR4-positive Vglut2 was determined. Consequently, as shown in Fig. 7B, the percentage of GR4-positive Vglut2 was significantly increased with CPTX administration. This result suggested that neural recombination occurred upstream of the injury site and produced new neural circuits.

Nonrecoverable wounds exist in spinal cord injury. It is probable that, during the CPTX-mediated recovery process from the spinal cord injury, CPTX may at least partly activate the reorganization of neural circuits, forming new neural circuits and allowing upstream and downstream contacts (see, e.g., Fig. 8). The suitability of CPTX for linking excitatory interneurons in the spinal cord may be associated with the sufficient expression of AMPA-type glutamate receptors in the excitatory interneurons.

### Description of sequence list

SEQ ID NO. 1: Nucleic acid and amino acid sequences of a fusion protein of the present invention prepared in the Example;
SEQ ID NO. 2: An amino acid sequence of a fusion protein of the present invention prepared in the Example {in SEQ ID NO. 2, the amino acid positions 1-28 are for the signal sequence, the amino acid positions 32-63 are for a Nrx-binding domain of Cblnl, the amino acid positions 66-98 are for a trimerization domain, the amino acid positions 110-317 are for an AMPA receptor-binding domain of aNPTX-1 protein, and the amino acid positions 321-326 are for a 6 × His tag};
SEQ ID NO. 3: An example of an amino acid sequence of a flexible linker;
SEQ ID NO. 4: An example of an amino acid sequence of a flexible linker;
SEQ ID NO. 5: An example of an amino acid sequence of a flexible linker;
SEQ ID NO. 6: An example of an amino acid sequence of a flexible linker;
SEQ ID NO. 7: An example of an amino acid sequence of a flexible linker;
SEQ ID NO. 8: An example of an amino acid sequence of a flexible linker;
SEQ ID NO. 9: An example of an amino acid sequence of a flexible linker;
SEQ ID NO. 10: A sequence of miRNA for the suppression of NP1 expression; and
SEQ ID NO. 11: A sequence of miRNA for the suppression of NPR expression.

## Claims

1. A multimer of one or more fusion proteins,
each fusion protein comprising a neurexin (Nrx)-binding region of a cerebellin-1 (Cbln1) protein, a multimerization domain, and a AMPA receptor-binding region of a neuronal pentraxin-1 (Nptxl) protein.

2. The multimer according to Claim 1, wherein the multimerization domain is a trimerization domain, and the multimer is a hexamer consisting of two trimers of the fusion protein, the trimers being linked to each other by a disulfide bond.

3. The multimer according to Claim 1 or 2, wherein the Nrx-binding region of the Cblnl protein is capable of binding to Nrx containing splice site 4 (i.e., Nrx(S4+)).

4. The multimer according to any one of Claims 1 to 3, wherein the an AMPA-type glutamate receptor-binding region of Nptxl corresponds to a pentraxin domain of human neuropentraxin-1.

5. The multimer according to any one of Claims 1 to 4, wherein the multimerization domain is interposed between the Nrx-binding region of the Cblnl protein and the AMPA-type glutamate receptor-binding region of Nptxl, or is sandwiched between the two regions.

6. The multimer according to any one of Claims 1 to 5, wherein the Nrx-binding region of the Cblnl protein and the multimerization domain are connected to each other via a linker and/or the multimerization domain and the AMPA receptor-binding region of Nptxl are connected to each other via a linker.

7. The multimer according to Claim 6, wherein the linker has one of a sequence of SEQ ID NOs. 3-9.

8. A nucleic acid encoding the fusion protein as defined in any one of Claims 1 to 5, wherein the nucleic acid has a nucleotide sequence of SEQ ID NO. 1.

9. The multimer according to any one of Claims 1 to 5, wherein the one or more fusion proteins has/have an amino acid sequence of SEQ ID NO. 2.

10. A pharmaceutical composition comprising the multimer according to any one of Claims 1 to 9.

11. A pharmaceutical composition for use in forming a new synaptic connection between excitatory interneurons,
the pharmaceutical composition comprising a fusion protein comprising a neurexin (Nrx)-binding region of a cerebellin-1 (Cbln1 protein), a multimerization domain, and a AMPA receptor-binding region of a neuronal pentraxin-1 (Nptxl) protein.

12. The pharmaceutical composition according to Claim 10 or 11, wherein the excitatory interneurons are those in the spinal cord.

13. The pharmaceutical composition according to any one of Claims 10 to 12, for use in treating a spinal cord injury.

14. The pharmaceutical composition according to any one of Claims 10 to 13, wherein the multimerization domain is a trimerization domain.

15. The pharmaceutical composition according to any one of Claims 10 to 14, wherein the multimerization domain is a trimerization domain, and the multimer is a hexamer consisting of two trimers of the fusion protein, the trimers being linked to each other by a disulfide bond.

16. The pharmaceutical composition according to any one of Claims 10 to 15, wherein the pharmaceutical composition is a therapeutic agent for a spinal cord injury.

17. A method for manufacturing the pharmaceutical composition according to any one of Claims 10 to 15, the method comprising:
culturing a cell having a nucleic acid having a signal sequence and encoding a fusion protein of the present invention under a condition suitable for protein expression;
acquiring a culture supernatant; and
collecting a multimer of one or more fusion proteins from the acquired culture supernatant.

18. The method according to Claim 17, wherein the multimerization domain is a trimerization domain, and the multimer is a hexamer consisting of two trimers of the fusion protein, the trimers being linked to each other by a disulfide bond.
